Europäisches Patentamt

European Patent Office

Office européen des brevets

(11) Publication number: **0 232 112 B1**

(12) **EUROPEAN PATENT SPECIFICATION**

(45) Date of publication of patent specification: **01.12.93**  (51) Int. Cl.5: **C12N 15/12**, C12P 21/02, C12N 5/00, A61K 35/16

(21) Application number: **87300695.1**

(22) Date of filing: **27.01.87**

(54) **Recombinant protein complex having human factor VIII:C activity, its production and use.**

(30) Priority: **27.01.86 US 822989**

(43) Date of publication of application:
**12.08.87 Bulletin  87/33**

(45) Publication of the grant of the patent:
**01.12.93 Bulletin  93/48**

(84) Designated Contracting States:
**AT BE CH DE ES FR GB GR IT LI LU NL SE**

(56) References cited:
**EP-A- 0 120 694**
**EP-A- 0 150 735**
**EP-A- 0 182 372**
**EP-A- 0 197 901**

**NATURE, vol. 312, no. 5992, November 1984, pp. 337-342, Reading Berks, GB; G.A. VEHAR et al.: "Structure of human factor VIII"**

**THE JOURNAL OF BIOLOGICAL CHEMISTRY, vol. 261, no. 27, 25. September 1986, pp. 12574-12578, American Society of Biological Chemists, Inc., US; R.L. BURKE et al.: "The functional domains of coagulation factor VIII:C"**

**NATURE, vol. 312, no. 5992, November 1984, pp. 330-337, Reading, Berks, GB; W.I. WOOD et al.: "Expression of active human factor VIII from recombinant DNA clones"**

(73) Proprietor: **CHIRON CORPORATION**
**4560 Horton Street**
**Emeryville, California 94608(US)**

Proprietor: **NOVO NORDISK A/S**
**Novo Allé**
**DK-2880 Bagsvaerd(DK)**

(72) Inventor: **Burke, Rae Lyn**
**1447 Willard Street**
**San Francisco, CA 94117(US)**
Inventor: **Rasmussen, Mirella Ezban**
**Abildgaardsgade 24**
**DK-2100 Copenhagen(DK)**

(74) Representative: **Patentanwälte Beetz sen. - Beetz jun. Timpe - Siegfried - Schmitt- Fumian- Mayr**
**Steinsdorfstrasse 10**
**D-80538 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Hemophilia A is an X-chromosome-linked inherited disease which afflicts 1-2 males per 10,000. The defect is manifest as a bleeding disorder due to the absence of clot formation arising from a lack of Factor VIII:C. Factor VIII:C has historically been isolated from blood in a concentrated form for therapeutic treatment of hemophilia. However, concerns about transmission of hepatitis and now AIDS have galvanized activity to provide alternative supplies of Factor VIII:C. It is of substantial interest to be able to supply compositions having Factor VIII:C activity without concerns as to the transmission viral diseases associated with the native Factor VIII:C.

Factor VIII:C is a very large glycoprotein (native $M_r$ 330-360 kiloDaltons (kD)), which is present in plasma at extremely low concentrations. The protein is very susceptible to cleavage by thrombin, plasmin, protease C, and other serine proteases. It is generally isolated from plasma or plasma products as a series of related polypeptides ranging from 160-40kD with predominant species of 92kD and 80-77kD. This complex pattern has made the analysis of the structure of active Factor VIII:C very difficult.

Rotblat et at., Biochemistry (1985) 24:4294-4300: Vehar et at., Nature (1984) 312:337-342; Toole et al., Nature (1984) 312:342-347; and Truett et al., DNA (1985) 4:333-349 describe Factor VIII:C and the related polypeptides. Orr et al., Molecular Genetics of Clotting Factors, p. 54, s321, reports a "spacer" function for the heavily glycosylated region of Factor VIII:C. Toole et al., supra; Wood et al., Nature (1984) 312:330-336; and Truett et at., supra, report sequencing of Factor VIII:C. Fulcher et al., Blood (1983) 61:807-811, report that the peak of maximum Factor VIII:C activity correlates with the presence of 90kD and 70kD fragments. Fulcher et al., J. Clin. Invest. (1985) 76:117-124, suggest that based on antibody-epitope data with Factor VIII:C, both the 92kD and the 80 kD polypeptides are necessary for Factor VIII:C function.

Although full-length recombinant human Factor VIII:C has been produced, it is difficult to purify and characterize, and it is unstable due to proteolysis. The practicality of being able to successfully produce and use the full-length molecule clinically is, therefore, doubtful at this time. Prior attempts to combine the 92kD and 80kD polypeptides in vitro did not produce an active composition. The present invention provides a means of making an active complex of the 92kD and 80kD chains.

It is the object of the present invention to provide a method of producing a recombinant protein complex having human Factor VIII:C activity but lacking all or part of the B domain of human Factor VIII:C, including the use of genes encoding the respective polypeptides, DNA compositions comprising these genes, and transformant host mammalian cells-comprising these DNA compositions.

The above object is achieved according to the independent claims. The dependent claims relate to preferred embodiments.

The method of the present invention for producing a recombinant protein complex having human Factor VIII:C activity but lacking all or part of the B domain of human Factor VIII:C comprises: co-expressing in trans in a eukaryotic transformant host cell

(a) a first gene encoding a signal sequence and a polypeptide having the amino acid sequence of the A domain of human Factor VIII:C or a sequence which differs therefrom by not more than 10% amino acid substitutions, and optionally including an N-terminal portion of the B domain of human Factor VIII:C, and

(b) a second gene encoding a signal sequence and a polypeptide having the same amino acid sequence of the C domain of human Factor VIII:C or a sequence which differs therefrom by not more than 10% amino acid substitutions, and

secreting the recombinant protein complex. As used herein the C domain includes A3 as described in Wood, et al., supra.

The DNA composition of the invention comprises first and second expression cassettes, said first expression cassette comprising a gene encoding a signal sequence and a peptide comprising the amino acid sequence of the A-domain of human Factor VIII:C or a sequence which differs therefrom by not more than 10% amino acid substitutions, and optionally an N-terminal portion of the B-domain of human Factor VIII:C under transcriptional and translational regulatory signals functional in a host cell, and said second expression cassette comprising a gene encoding a signal sequence and a peptide comprising the sequence of the C-domain of human Factor VIII:C or a sequence which differs therefrom by not more than 10% amino acid substitutions,and not including the B-domain under transcriptional and translational regulatory signals functional in said host cell.

Another aspect of the invention is to provide a host mammalian cell containing the above-described DNA composition.

The invention also generally provides the use of genes encoding polypeptides having the N-terminus and the C-terminus of a Factor VIII:C protein complex, each of said genes having an independent signal sequence, in an expression system for producing a protein complex having Factor VIII:C activity.

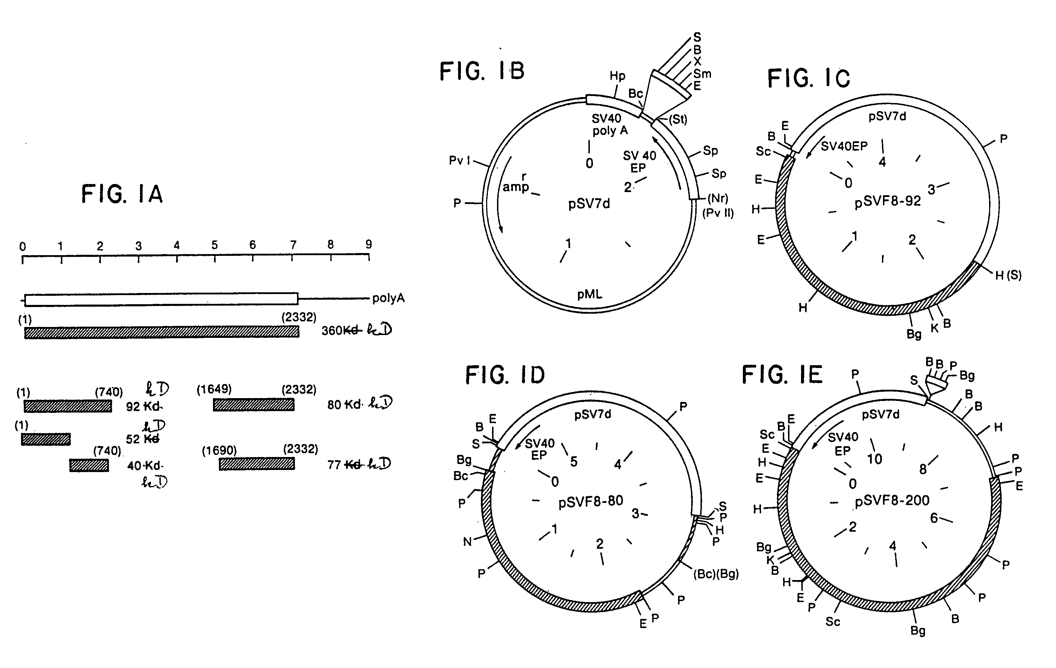

Fig. 1A depicts the location of the proteolytic cleavages responsible for the generation of the 92 kD and 80 kD chains.

Figs. 1B-E indicate selected restriction maps for the plasmids pSV7d, pSVF8-92, pSVF8-80 and pSVF8-200, the source or function of fragments and the number of nucleotides starting from the point indicated by 0. The selected restriction sites are: B, BamHI; Bc, BclI; Bg, BglII; E, EcoRI; H, HindIII; Hp, HpaI; K, KpnI; N, NdeI; Nr, NruI; P, PstI; Pvul, PvuI; PvuII, PvuII; S, SalI; Sc, SacI; Sm, SmaI; St, StuI; X, XbaI. Those sites enclosed within parentheses were used in the vector construction, but were not regenerated.

The protein compositions obtained in accordance with the above methods have Factor VIII:C activity. The compositions comprise two chains one chain having the same or substantially the same amino acid sequence as the N-terminal portion (A domain) of Factor VIII:C, and the other chain having the same or substantially the same amino acid sequence as the C-terminus (C domain) of the Factor VIII:C protein as defined above. The two chains are about 92 kD for the A domain (except when extended into the B domain) and 80kD for the C domain, and are sometimes referred to, respectively, as the "heavy" chain and the "light" chain. Nucleic acid constructs are provided comprising individual expression cassettes having transcriptional and translational regulatory regions functional in a mammalian cell, which control the transcription and the translation of the structural genes, where each structural gene has a sequence encoding for the mature peptide chain joined at its N-terminus to a signal sequence. The expression cassettes are introduced into a mammalian host, whereby a protein complex is produced having Factor VIII:C activity. (In referring to the sequences and domains of Factor VIII:C, the sequences and domains referred to are found on p. 333 of Wood et al. cited above.)

The N-terminal polypeptide will extend from amino acid 10, usually amino acid 1, to at least about amino acid 620, usually at least about amino acid 675, more usually at least about amino acid 740. The polypeptide will include at least about 85% of the A domain (Wood et al., supra), more usually at least about 90%, and may optionally include a portion of the N-terminus of the B domain, typically not exceeding about amino acid 1405. Of particular interest is an N-terminal chain having the entire sequence to the thrombolytic cleavage site at $Arg_{740}$-$Ser_{741}$.

The light chain will have an amino acid sequence substantially the same as the amino acid sequence of the C-terminus of a Factor VIII:C polypeptide, usually at least about 80%, more usually at least about 90% of the Factor VIII:C 80kD chain, particularly beginning with amino acid 1570, usually amino acid 1600, particularly amino acid 1625, more particularly amino acid 1640, preferably at about amino acid 1649, ±10 amino acids, more particularly ±1 amino acid, and continuing to at least about amino acid 2300, usually 2310, ±10 amino acids, preferably 2325, ±5 amino acids, more preferably to the terminal amino acid (2332). Usually, the light chain will have at least about 85%, more usually at least 95%, of the C1-C2 domains, desirably the A3-C1-C2 domains.

Usually not more than 10, more usually not more than 5 number%, and preferably not more than about 1 number% of the amino acids in the chains will differ from the amino acids naturally present in the Factor VIII:C A and C domains. Particularly, not more than about 5%, and more usually not more than about 1% will be nonconservative substitutions. Conservative substitutions include: G,A; V,I,L; D,E; K,R; N,Q; F,W,Y, where the letters between the semicolons are conservative substitutions. (The letters are one-letter code for amino acids.) Where an amino acid in one grouping is substituted by an amino acid in another grouping or an amino acid which is not indicated above (C,M,H,P) such change will be considered nonconservative.

DNA constructs will be employed for expression of the indicated polypeptides. Each of the constructs will have in the 5'-3'-direction of transcription a transcriptional initiation and translational initiation region, a structural gene coding region comprising a sequence coding for the peptide signal sequence, including the processing signal, and a sequence coding for the Factor VIII:C heavy or light chains, followed by the translational and transcriptional termination region.

The initiation region may be comprised of a number of different sequences involved with the initiation of transcription and translation. These sequences include enhancer sequences, RNA polymerase binding site, capping site, ribosomal binding and translational initiation sites, and the like. The transcriptional initiation region may be the natural or wild type region associated with Factor VIII:C or may be alternative region to provide for high transcriptional efficiency. The region may be obtained from viruses having mammalian host ranges or the genes of the host cell or genes from a different mammalian host compatible with the host cell. Numerous transcriptional initiation regions have been isolated and demonstrated to be operative in mammalian hosts. These regions include the SV-40 early promotor and late promoter regions, the adenovirus major late promoter region, β-actin promoter region, the cytomegalovirus 72kD early protein promoter region, the metallothionein promoter, and the like.

The termination region may include the polyadenylation signal sequence, the transcriptional termination sequence, and the like. The termination region may be obtained from the 3′-region of the Factor VIII:C natural or wild type region, or may be from the same structural gene or different structural gene from which the 5′-initiation region was obtained. The 3′-region is not as essential to the level of transcription as the initiation region, so that its choice would be more of a matter of convenience than specific selection.

The structural genes will consist of a signal sequence which codes for the N-terminal amino acid sequence, and which directs the polypeptide into the lumen of the endoplasmic reticulum for processing and maturation. Also included in the signal sequence is the processing signal which is recognized by an endopeptidase, where the endopeptidase cleaves a peptide bond, removing the signal sequence to provide the mature polypeptide. The signal sequence may be the naturally occurring signal sequence, particularly for the N-terminal peptide or may be any signal sequence which is operative with the peptides to provide for the processing and maturation of the polypeptides.

Various signal sequences have been reported in the literature and include such signal sequences as tissue plasminogen activator, immunoglobin heavy and light chains, viral membrane glycoproteins such as Herpes Simplex virus glycoproteins gB and gD, and the like.

When necessary, the sequence encoding the mature protein and signal sequence will be joined so as to be in reading frame. Where convenient restriction sites are available, the cohesive or blunt ends may be properly joined. However, for the most part, adaptors will be employed where portions of the coding sequence will be recreated in the synthetic adaptor so that the truncated structural gene and/or truncated signal sequence will be linked through the adaptor, so as to be in proper reading frame. The signal sequence and structural gene may be partially restriction mapped, so as to identify restriction sites, particularly unique restriction sites, which may be employed to link the two sequences together in proper reading frame by means of an appropriate adaptor. Alternatively unique restriction sites may be inserted at the signal sequence recognition site by in vitro mutagenesis.

The translational start and stop signals will normally be part of the structural gene, providing for the appropriate initiation codon at the beginning of translation and one or more stop codons at the termination of translation. These codons will frequently be present in the sequences as employed, particularly the signal sequences providing the initiation codon. The stop codons may be added as appropriate as part of the termination region or be added to the coding region to provide for convenient 3′-terminus for linkage to the transcriptional termination region to provide for a complete termination region.

The various regions of the expression cassette, (the transcriptional and translational initiation region nucleic acid sequence, structural gene nucleic acid sequence encoding one of the polypeptides and under the transcriptional and translational control of the initiation region, and a transcriptional and translational termination region, controlling the processing of the mRNA and the translational termination) which identify the particular nucleotide sequences may be joined using conventional methods. Usually, the sequences obtained will contain, or be modified to contain restriction sites, which may then be annealed, where complementary overhangs or cohesive ends are present. Modification frequently will be in noncoding regions by the introduction of linkers to provide for the desired cohesive ends. The ends will usually be ligated prior to introduction into the host cell, although the host cell may be allowed to provide the necessary ligation.

The expression cassettes may be joined to a wide variety of other sequences for particular purposes. Where amplification is desired, the expression cassettes may be joined in tandem to a gene which is amplified when induced by an appropriate stimulus. Such genes as the metallothionein genes, e.g., human metallothionein gene, dihydrofolate reductase, and mouse mammary tumor virus LTR may be joined to the peptide cassettes having their own transcriptional and translational regulatory sequences. By employing heavy metal ions, e.g., copper or cadmium, methotrexate, or glucocorticoids, amplification of the amplifying gene and the gene of the interest (the expression cassette) may be achieved in the host cell. The structural genes will have the appropriate sequences for expression as described for the expression cassette.

The subject expression cassettes may be joined to a vector, which will comprise a replication system functional in the host cell, which replication system may provide for stable episomal maintenance or integration of the expression cassette into the host genome. The vector will also comprise a marker for selection, for selecting host cells containing the DNA construct and the vector from those host cells which lack the DNA construct and vector.

A wide variety of replication systems are available, normally from viruses that infect mammalian host cells. Illustrative replication systems include the replication systems from Simian virus 40, adeno-virus, bovine papilloma virus, polyoma virus, Epstein-Barr virus, and the like.

Markers may include resistance to a biocide, particularly an antibiotic, or complementation of auxotrophy to provide a prototrophic host. Particular genes of interest as markers include kanamycin resistance gene (NPTII), chloramphenicol resistance gene (CAT), penicillinase, or the like.

Usually, the vector will be circular, and will have one or more restriction sites which allow for the insertion of the expression cassette, stepwise or as a completed entity, into the vector. Frequently, the vector will also include a bacterial replication and selection system, which allows for cloning after each of the manipulative steps. In this way, relatively large amounts of the construction at each of the stages may be prepared, isolated, purified, checked to see that the proper joining has occurred, and then used for the next stage.

Various mammalian host cells may be employed in which the regulatory sequences and replication system are functional. Such cells include COS cells. Chinese hamster ovary (CHO) cells, mouse kidney cells, hamster kidney cells, HeLa cells, HepG2 cells, or the like.

The expression cassettes of the desired polypeptides may be linked together in one nucleic acid chain or may be present in separate nucleic acid molecules. Conveniently, the expression cassettes may be parts of different vectors or of the same vector. This is primarily a matter of convenience, although in some situations with particular vectors, one or the other manner of construction may be desirable.

The manner in which the expression cassettes are introduced into the host cell will be conventional. Conveniently, calcium phosphate precipitated DNA or DNA in the presence of DEAE-dextran may be employed for transformation. Where viruses are involved, transfection or transduction may be employed. The particular manner in which the host cell is transformed is not critical to this invention, depending substantially upon whether the expression cassettes are joined to a replication system and the nature of the replication system and associated genes.

The transformed cells may then be grown in an appropriate nutrient medium. The product is obtained as a complex of the two chains, so that the media or cell lysate may be isolated and the Factor VIII:C active complex extracted and purified. Various means are available for extraction and purification, such as affinity chromatography, ion exchange chromatography, hydrophobic chromatography, electrophoresis, solvent-solvent extraction, selective precipitation, and the like. The particular manner in which the product is isolated is not critical to this invention and will be chosen to minimize denaturation or inactivation and maximize the isolation of a high purity active product.

Compositions are provided where the composition in the Coatest will have at least 0.02U/ml of activity, usually at least about 0.2, and more usually at least about 0.5U/ml of activity. The subject product can be purified by affinity chromatography using antibodies, particularly monoclonal antibodies directed against the C-terminus subunit of the Factor VIII:C activity, electrophoresis, extraction, HPLC, etc.

The subject method provides for production of a complex of the heavy and light chains which has Factor VIII:C activity. Production is evidenced by conditioned media as described in the experimental section, which will have at least about 50, usually at least about 70mU/ml, more usually at least about 200mU/ml of Factor VIII:C activity in the Coatest assay.

The complexes having Factor VIII:C activity produced according to the invention have a variety of uses as immunogens for the production of antibodies, for isolation of von Willebrand factor by affinity chromatography, in diagnostic assays for Factor VIII:C and for treatment of hemophiliacs and other hosts having blood clotting disorders. The subject protein complexes may be administered in a physiologically acceptable carrier, such as water, saline, phosphate buffered saline, and citrate buffered saline, at concentrations in the range of about 10-200U/ml. See US 3,631,018, 3,652,530, and 4,069,216 for methods of administration and amounts. Other conventional additives may also be included.

The following examples are offered by way of illustration and not by way of limitation.

Experimental

Example 1

A. Preparation of Expression Plasmids

A.1 pSV7d: A mammalian cell expression vector

The expression cassettes were prepared using the mammalian cell expression vector pSV7d (2423bp).

The plasmid pSV7d (see Truett et al., supra) was constructed as follows: The 400bp BamHI/HindIII fragment containing the SV40 origin of replication and early promoter was excised from pSVgtl (Gruss, P., and Khoury, G., Proc. Natl. Acad. Sci. USA (1981) 78:133-137) and purified. The 240bp SV40 BclI/BamHI fragment containing the SV40 polyA addition site was excised from pSV2/DHFR (Subramani et al., Molec. and Cell Biol. (1981) 1:854-864) and purified. The fragments were fused through the following linker:

## Stop Codons

```
                       1    2    3
5'-AGCTAGATCTCCCGGGTCTAGATAAGTAAT-3'
   TCTAGAGGGCCCAGATCTATTCATTACTAG
```

__Hind__III  __Bgl__II __Sma__I  __Xba__I        __Bcl__I overhang.

This linker contains five restriction sites, as well as stop codons in all three reading frames. The resulting 670bp fragment containing the SV40 origin of replication, the SV40 early promoter, the polylinker with stop codons and the SV40 polyadenylation site was cloned into the BamHI site of pML, a pBR 322 derivative with about 1.5kb deletion (Lusky and Botchan, Cell (1984) 36:391), to yield pSV6. The EcoRI and EcoRV sites in the pML sequences of pSV6 were eliminated by digestion with EcoRI and EcoRV, treated with Bal31 nuclease to remove about 200bp on each end, and finally religated to yield pSV7a. The Bal31 resection also eliminated one BamHI restriction site flanking the SV40 region, approximately 200bp away from the EcoRV site. To eliminate the second BamHI site flanking the SV40 region, pSV7a was digested with NruI, which cuts in the pML sequence upstream from the origin of replication. This was recircularized by blunt end ligation to yield pSV7b.

pSV7c and pSV7d represent successive polylinker replacements. First, pSV7b was digested with StuI and XbaI. Then, the following linker was ligated into the vector to yield pSV7c:

__Bgl__II __Eco__RI   __Sma__I    __Kpn__I __Xba__I

```
5'-AGATCTCGAATTCCCCGGGGGTACCT
   TCTAGAGCTTAAGGGGCCCCCATGGAGATC
```

Thereafter, pSV7c was digested with BqIII and XbaI, and then ligated with the following linker to yield pSV7d:

__Bgl__II __Eco__RI    __Sma__I __Xba__I  __Bam__HI __Sal__I

```
5'-GATCTCGAATTCCCCGGGTCTAGAGGATCCGTCGAC
   AGCTTAAGGGGCCCAGATCTCCTAGGCACGTGGATC
```

A.2 pSVF8-92: An expression plasmid for the 92kD chain

Starting from the BamHI site in the polylinker pSV7d, pSVF8-92 consists of a 49bp synthetic linker-adaptor molecule from BamHI to SacI encoding nucleotides -30 to +14 of the Factor VIII:C protein, (numbering from the first A of the translational start site; the sequence is shown below in A.4) a 2267bp SacI to HindIII fragment from the Factor VIII:C DNA contained in pSVF8-200 described below (up to nucleotide +2281), and pSV7d from HindIII to BamHI.

A.3 pSVF8-80: An expression plasmid for the 80kD chain

Starting from the SalI site in the polylinker pSV7d, pSVF8-80 consists of a 201bp fragment of a tissue plasminogen activator cDNA from nucleotides -98 to +103 (relative to the start codon) terminating at a BgIII site (tPA sequences given in Degan, S.J.F., et al., J. Biol. Chem. (1986) 261:6972-6985), a 29bp synthetic BgIII to BcII linker-adaptor encoding nucleotides +5002 to +5031 of Factor VIII:C ligated to a 2464bp BcII fragment of factor VIII:C spanning from a BcII site created at nucleotide 5028 of the factor VIII:C cDNA through in vitro mutagenesis (Zoller and Smith, Methods in Enzymology (1983) 100:468), to a BcII site in the 3' untranslated region, at nucleotide 7492, and a 400bp fragment of tPA 3' untranslated sequence spanning from a BglII site to a synthetic PstI site generated from the cDNA cloning, followed by the

6

polylinker from the vector M13mp9 (Vieira and Messing, Gene (1982) 19:259) and then pSV7d.

A.4 pSVF8-200: An expression plasmid for the full length Factor VIII:C cDNA

The plasmid pSVF8-200 (described in Truett et al.), which contains the entire Factor VIII:C cDNA coding and 3' untranslated sequences, with the 5' untranslated sequences the same as described above for pSVF8-92, was prepared as follows.

Plasmid pSV7d was digested with BamHI to cut in the polylinker region downstream of the SV40 early promoter. The following 49bp BamHI-SacI linker adaptor, which codes for the last 30bp of the 5' untranslated region and the first 15bp of the human Factor VIII:C coding sequence, was chemically synthesized and ligated to pSV7d:

```
           -35    -30    -25    -20    -15    -10    -5        met gln ile glu
   5'    GATCC  TCTCC  AGTTG  AACAT  TTGTA  GCAAT  AAGTC  ATG  CAA  ATA  GAG  CT  3'
   3'  BamHI G  AGAGG  TCAAC  TTGTA  AACAT  CGTTA  TTCAG  TAC  GTT  TAT  CSacI     5'
```

This ligated plasmid was subsequently digested with SacI to remove excess linkers and with SalI to provide a SalI overhang.

Fragment 1, the 2.9kb SacI fragment from pF8-102 containing the 5' coding region of human Factor VIII:C, and Fragment 2, the 6.5kb SacI-SalI fragment from pF8-6.5 which contains the 3' coding region of the factor, and pSV7d modified vector containing the linker adaptor were ligated together (see Truett et al., supra). This ligation mix was then used to transform E. coli HB101, and colonies were selected by resistance to ampicillin.

Three hundred transformants were screened by colony filter hybridization using the BamHI-SacI 5' adaptor or the 2.9kb SacI fragment as probes. Those colonies positive with both probes were then analyzed by restriction mapping. Plasmid pSVF8-200, which contains the entire coding region for the human Factor VIII:C gene and a 5' untranslated region properly fused in transcriptional orientation to the SV40 early promoter, was obtained.

B. Transfection and Culture of COS7 Cells

The plasmids described above were transfected into COS7 cells (Guzman, Cell (1981) 23:175) using the calcium phosphate coprecipitation method (van der Eb and Graham, Meth. Enzymology (1980) 65:826-839) coupled with treatment with chloroquine diphosphate (Luthman and Magnusson, Nucl. Acids Res. (1983) 11:1295-1308) using 50μg of plasmid DNA per 5x10⁵ cells for 14hr. Cells may also be transfected by the DEAE-dextran method of Sompayrac and Danna P.N.A.S. (1981) 78:7575-7578.

The COS7 cells were cultured in Dulbecco's modified Eagle medium supplemented with 10% fetal calf serum, 100U/ml penicillin, 100μg/ml streptomycin, 292μg/ml glutamine, and 110μg/ml sodium pyruvate. Samples were obtained from a 48-hour collection of serum containing medium at 88 hours post transfection.

C. Assays

At specific intervals post transfection, media was removed from the cells, and aliquots were stored at -70°C. Samples were tested for their ability to decrease the prolonged partial thromboplastin time of Factor VIII:C deficient plasma in a standard coagulation assay (Hardisty et al., Thrombosis et Diathesis Haemologica (1962) 72:215). The more specific Coatest assay (Rosen et al. in Thromb. and Haemostasis (1985) 54:818-823), which measures the generation of activated Factor X (Xa) as a linear function of the concentration of exogenously supplied Factor VIII:C, was used to verify the results of the coagulation assay. The concentration of immunologically reactive Factor VIII:C protein in the medium was determined by the application of a radioimmunoassay (RIA) developed to detect the 92kD polypeptide and by an enzyme-linked immunosorbant assay (ELISA) specific for the 80kD polypeptide (Nordfang et al., Thromb. and Haemostasis (1985) 53:346).

As shown in Table 1, expression of the 92kD polypeptide or of the 80kD polypeptide alone produced no detectable activity even though high levels of each of the individual proteins were present in the conditioned media. When cells were cotransfected with pSVF8-92 and pSVF8-80 plasmids, the media contained about 20mU/ml of coagulation activity. The same relative level of the coagulation activity was secreted by cells transfected with the plasmid pSVF8-200 encoding the complete Factor VIII:C protein.

When conditioned media from the pSVF8-92 and the pSVF8-80 single transfectants were mixed together (using several different conditions as outlined in Table 1) no activity was measurable.

These results indicate that a complex of the amino and carboxyl terminal domains of Factor VIII:C retains intrinsic coagulation activity and that the interior B domain is not essential for activity nor for the assembly of an active complex from separate chains.

TABLE 1: Assay of Recombinant Factor VIII:C Activity in Conditioned COS Cell Media

| | ELISA[d] Assay | Coagulation Activity[a] | | Coatest[b] Activity | RIAC Assay | |
|---|---|---|---|---|---|---|
| Plasmid | U/ml | Coagulation Time (s) | (mU/mle) | (mU/ml) | 92kD (U/ml) | 80kD |
| pSVF8-92 | <.0002 | 95.7 | < .9 | < .1 | 0.15 | |
| pSVF8-80 | 1.36 | 97.2 | < .9 | < .1 | <.01 | |
| pSVF8-92 +pSVF8-80 | 1.13 | 56.1 | 22.5 | 20.4 | 0.05 | |
| pSVF8-200 | 0.28 | 47.7 | 70.0 | 43.2 | 0.12 | |
| none | <.0002 | 94.6 | < .9 | < .1 | <.01 | |
| pSVF8-92) mixed in vitro[f] pSVF8-80 | | 95.7 | < .9 | < .1 | -- | |

a  Aliquots of 75µl of media conditioned by the growth of COS cells transfected with the indicated plasmids or mock transfected were assayed for their ability to decrease the prolonged partial thromboplastin time of Factor VIII:C deficient plasma in the one-stage assay. Briefly, 75µl of Platelin (General Diagnostics) was incubated for 3min at 37°C followed by the addition of 75µl of Factor VIII:C deficient plasma plus 75µl of the test sample for an additional 5min incubation at 37°C. A 75µl aliquot of prewarmed 0.025M CaCl₂ was added and the clotting time measured with a Becton-Dickinson fibrometer. Normal human plasma diluted in COS cell medium was used as a standard.

8

TABLE 1 (continued) . . .

b   The Coatest assay (Kabi) measures the generation of activated Factor X (Xa) as a linear function of the concentration of Factor VIII:C. The concentration of Factor Xa is measured by the proteolytic cleavage of the chromogen para-nitroaniline from a synthetic peptide substrate for Xa. Normal human plasma diluted in 50mM Tris-HCl, pH 7.3, 0.2% BSA was used as the standard.

c   For the RIA assay purified dog factor VIII:C inhibitory IgG was coated onto the wells of a 96-well polystyrene microtiter plate at a concentration of 3.5 µg/ml in 0.1 M sodium carbonate buffer, pH 9.8, by overnight incubation at 37°C. The plates were washed 3 times with 0.1 M NaCl, 0.05% Tween 20 followed by an incubation with a mixture of test medium samples and iodinated VIII:C 92-kD protein, both diluted in 0.05 M imidazole, 0.1 M NaCl, 1% bovine serum albumin, 0.05% Tween 20, pH 7.3. The VIII:C 92-kD protein was isolated from plasma and was greater than 50 percent homogenous as estimated by sodium dodecyl sulfate polyacrylamide gel electrophoresis and silver staining. After incubation for 16 h at room temperature, the plates were washed, and the amount of $^{125}$I in the individual wells was measured in a γ counter. An intermediate purified commercial factor VIII:C preparation (factor VIII, NORDISK) with a specific activity of 0.5 unit of coagulation activity per mg was used as the standard. This standard was calibrated against the World Health Organization Third International factor VIII:C standard. We defined our intermediate purified standard to contain a 92-kD RIA activity/factor VIII:C coagulation activity ratio of 1.

d   For the ELISA assay, purified human Factor VIII:C inhibitory IgG was coated onto the wells of a 96-well polyvinylchloride microtiter plate at a concentration 4.5µg/ml in 0.1M sodium carbonate, pH 9.8, by overnight incubation at 37°C. The wells were washed as above and peroxidase-conjugated F(ab)'$_2$ fragments of the human inhibitory IgG diluted in 0.1M imidazole, 0.15M NaCl, 1% BSA, 0.05% Tween 20, pH 7.3, were added for a final incubation of 16h at room temperature. The color was developed with o-phenylenediamine solution. Normal human serum was used as a standard.

e   One mU of activity is assumed to correspond to approximately 100pg of Factor VIII:C protein (Fay et al., Proc. Natl. Acad. Sci. USA (1982) 79:7200).

f   A variety of mixing conditions were tested including preincubation for various times up to 2h at 37°C, 20°C, or 4°C in the presence or absence of 10mM CaCl$_2$. The value reported in this table is representative of the data obtained.

EP 0 232 112 B1

TABLE 2:   Coagulation Inhibition Assay

### Experiment I:

| Plasmid | IgG[a] | Coagulation Time (s) |
|---|---|---|
| pSVF8-80 + pSVF8-92 | N | 51.9 |
|  | I | 74.5 |
|  | B | 54.4 |
| pSVF8-200 | N | 46.4 |
|  | I | 69.4 |
|  | B | 46.8 |

For Experiment I, 160μl of the indicated COS cell conditioned media was incubated with 20μl of a 100-fold dilution of human Factor VIII:C inhibitory serum (Bethesda titer 1500 units) or a similar dilution of pooled norman human serum, or buffer alone (50mM imidazole, 0.1M NaCl, 100μg/ml BSA pH 7.3) for 2h at 37°C.   These samples were then assayed for residual coagulation activity as outlined in the legend to Table 1.

### Experiment II:

| Plasmid | IgG[a] | Coagulation Time (s) |
|---|---|---|
| pSVF8-92 + pSVF8-80 | I | 72.9 |
|  | B | 48.0 |
| pSVF8-200 | I | 60.9 |
|  | B | 44.9 |

For Experiment II, 100μl of conditioned media was incubated for 2h at 37°C with either 10μl of a 1μg/μl solution of anti-Factor VIII:C monoclonal antibody from Hybritech (Bethesda titer 14,000 units) or buffer and then assayed as above.

a
Normal serum = N; inhibitory serum = I; buffer = B.

To verify that the observed coagulation activity was due to Factor VIII:C, the sensitivity of the coagulation to inhibition by an antibody specific for Factor VIII:C was determined. Prior to assay, aliquots of conditioned media were preincubated for 2hr at 37°C in the presence of dilutions of normal human serum or of serum from a hemophiliac who had developed a high titer of inhibitory antibodies to Factor VIII:C. As shown in Table 2, the activity of the complete molecule, as well as that of the 92kD-80kD complex was reduced specifically by the inhibitory serum. The same results were obtained using three different inhibitory monoclonal antibodies which bind to the 80kD species.

To demonstrate more clearly the existence of a two chain complex, the active species was partially purified from the COS cell media by passage over a monoclonal antibody column directed against the 80kD portion as shown in Table 3. Approximately 65% of the applied activity was retained by the column and 50% of this bound material was eluted in an active form and at a fivefold greater concentration than the initial media. Thus an active complex can be isolated by affinity chromatography using an antibody specific for only the 80kD species.

TABLE 3:   Partial Purification of 92kD-80kD Coagulation Active Complex

| Fraction | Coatest $\left(\text{U/ml}\right)$ | 80kD ELISA $\left(\text{U/ml}\right)$ |
|---|---|---|
| Media | .0044 | 0.175 |
| Flowthrough | .0017 | 0.13 |
| Eluate | .0200 | 0.76 |

100µg of an anti-80kD monoclonal antibody (56 IgG) (Nordfang et al., Thromb. Haemostasis (1985) 53:346) coupled to Sepharose CL4B was incubated overnight at 20°C with 1.4ml of media containing a total of 6.2mU of activity (measured by the Coatest Assay obtained from COS cells cotransfected with pSVF8-92 and pSVF8-80 plasmids). After incubation, the slurry was loaded into a column and the flowthrough fraction was collected. The column was washed with 300µl of Buffer A (50mM imidazole, .1M NaCl, 0.1% sodium insulin, 0.2% NaN$_3$, pH 7.3) and then eluted with 300µl of Buffer B (2.5M NaCl, 50% ethylene glycol, 0.5M imidazole, 0.1M CaCl$_2$, 0.1% sodium insulin, 0.2% NaN$_3$, pH 7.3).

Results reported here demonstrate that expression of the $\beta$ linker region, containing 918 amino acids or about 40% of the total for the intact protein, is not required for Factor VIII:C activity. Co-expression of individual 92kD and 80kD regions results in a level of Factor VIII:C activity comparable to that obtained from the expression of the whole Factor VIII:C coding region. These proteins assemble in vivo to form an active complex linked by a calcium bridge. The assembly does not require the presence of the $\beta$ region and occurs efficiently for the two chains expressed in trans.

It is evident from the above results that Factor VIII:C activity can be achieved by directly producing an N-terminal fragment and a C-terminal fragment which are independently expressed, each having its own signal sequence. Thus, Factor VIII:C can be obtained more efficiently, since the large precursor need not be cloned and used as the coding sequence for the Factor VIII:C activity. Thus, cells may be employed for expression of Factor VIII:C which may be deficient in the capability of the proper maturation of the Factor VIII:C protein.

Example 2

Expression of the 92 kD protein in COS cells using the pSVF8-92 construction was low compared to the amount of 80kD protein produced. Accordingly, the construction was modified in an attempt to increase the level of 92kD protein. Modifications of the following types were made: Changes in the 5′ untranslated sequence of the Factor VIII:C gene; inclusion of heterologous 5′ untranslated and leader sequences; and

11

changes in the 3′ untranslated sequences. These constructs are summarized below.

A. Expression Plasmids

A.1 5′ Untranslated Region Modifications Plasmid pSVF8-92B. This plasmid is a derivative of pSVF8-92 in which the 30bp of 5′ untranslated sequence of pSVF8-92 is replaced with the entire 5′ untranslated region of human Factor VIII:C cDNA (nucleotides 1 to 171; see Fig. 8 of Truett et al., supra), with a deletion of the G-C tails (by in vitro site-specific mutagenesis), and the three base changes shown below at the starting ATG (at position +172, Fig. 8, Truett, et al., supra) to conform to Kozak's preferred sequences for efficient message translation in eukaryotic cells:

Factor VIII:C: GTCATG CAA

Kozak consensus: ACCATG G

This change alters the second amino acid of the signal peptide to Glu from Gln.

Plasmid pSVF8-92E. This plasmid is a derivative of pSVF8-92B in which the polylinker derived from pSV7d 5′ to the Factor VIII:C sequences is removed with the exception of the SalI site, and the ATG codon in the 5′ untranslated region (at 41 according to Truett et al., supra) is altered to ATT, by in vitro mutagenesis.

A.2 Addition of Heterologous 5′ Untranslated Regions and Leader Sequences

Plasmids pSVF8-92G, H, and I. These plasmids are derivatives of pSVF8-92B in which the 5′ untranslated region as well as the natural Factor VIII:C signal sequences are replaced with the analogous region from the human tissue plasminogen activator (tPA) cDNA gene. In pSVF8-92G the first 35 amino acids (signal sequence) of the tPA 5′ region are joined to mature Factor VIII:C 92 kD with a serine substituted for the first amino acid (alanine) of the 92 kD protein. In pSVF8-92H the first 32 amino acids of the tPA 5′ region are joined to mature Factor VIII:C 92 kD protein. In pSVF8-92I, the first 23 amino acids of the tPA 5′ region are joined to mature Factor VIII:C 92 kD protein. The tPA sequences are the same as those described for pSVF8-80.

Plasmid pSVF8-92J. This plasmid is a derivative of pSVF8-92G in which the tPA 5′ region is replaced with 75bp of herpes simplex virus -1 (HSV-1) gD 5′ untranslated sequences and 75bp of HSV-1 gD signal sequence. pSVF8-92J also lacks the Ala → Ser substitution (Watson, R.J., et al., Science (1982) 218:381-384).

A.3 3′ Untranslated Region Changes

Plasmid pSVF8-92C. This plasmid is a variation of pSVF8-92B in which the 92kD coding region is fused directly to the translational stop codon and natural 3′ untranslated sequences of human Factor VIII:C cDNA. This plasmid is a derivative of pSVF8-200.

Plasmid pSVF8-92L. This plasmid is a derivative of pSVF8-92C in which the 3′ untranslated region of pSVF8-92C is replaced with the 3′ untranslated region of pSVF8-80.

B. Results

Each of the plasmids of part A was transfected into COS7 cells along with pSVF8-80 as described in Example 1 and the media tested for Factor VIII:C activity as in Example 1.

pSVF8-92B, the first tested, showed activity levels ranging from 2-to-8-fold better than pSVF8-92. Of the remaining plasmids pSVF8-92E appeared to be the best, being 1.65-fold better than pSVF8-92B. pSVF8-92J and I also produced substantially higher expression levels than pSVF8-92, being close to that of pSVF8-92E. The expression level of pSVF8-92G approximated that of pSVF8-92, whereas that of pSVF8-92H was substantially less than pSVF8-92. The expression levels of both pSVF8-92C and pSVF8-92L appear to be equivalent to that of pSVF8-92E.

Example 3

This example describes the preparation of constructs for producing polypeptides that consist of the 92 kD chain and a portion of the B domain. These derivatives were made in an attempt to develop a heavy chain that is more stable and/or assembles more efficiently into an active complex with the light chain. The derivatives were chosen to mimic molecular species that have been observed in plasma-derived preparations of Factor VIII:C and in cell lysates and conditioned media from cells expressing recombinant full-length Factor VIII:C. These could possibly arise by thrombin cleavages of full-length Factor VIII:C.

A. Preparation of Expression Plasmids

A.1 pSVF8-92S

This plasmid encodes a 982 amino acid heavy chain and was prepared from a full-length cDNA plasmid pSVF8-302 by partial cleavage at the first SacI site of the B-domain coding region. An oligonucleotide adaptor was used to install a translational stop codon and fuse the coding sequence to the natural human Factor VIII:C 3′ untranslated sequence beginning at the first BalI site. This plasmid encodes the first 978 amino acids of native human Factor VIII:C and 4 substituted amino acid residues at the carboxy terminus.

A.2 pSVF8-160

This plasmid provides a 1323 amino acid heavy chain and was prepared from a full-length clone (designated pSVF8-303) similar to pSVF8-200, but having the 5′ untranslated region of pSVF8-92E. pSVF8-303 was cleaved with EcoRV and SmaI, and the blunt ends were ligated together to form pSVF8-160. This plasmid encodes the first 1315 amino acids of Factor VIII:C. Eight nonsense amino acids are added at the carboxyl terminus as a result of the fusion of the polylinkers of the vector pSV7d.

A.3 pSVF8-170

This plasmid provides a 1416 amino acid heavy chain and was also prepared from pSVF8-303. pSVF8-303 was partially digested with BglII, and the resulting 6811 bp fragment was gel isolated and the ends ligated together to form pSVF8-170. This plasmid encodes the first 1405 amino acids of Factor VIII:C and has a carboxyl extension of 11 nonsense amino acids due to fusion of the polylinker of the vector pSV7d.

A.4 pSVF8-120

This plasmid provides a 1107 amino acid heavy chain and was prepared from pSVF8-303. The plasmid pSVF8-303 was digested with ApaI and the cohesive ends were filled in with T4 polymerase. The resulting molecule was further digested with SmaI, the DNA self-ligated and propagated in E. coli HB101. This plasmid encodes 1102 amino acids from the amino terminus of Factor VIII:C plus an additional 5 nonsense amino acids at the carboxyl terminus.

B. Results

Each of the plasmids of part A was transfected into COS7 cells along with pSVF8-80 as described in Example 1 and the media tested for Factor VIII:C activity as in Example 1.

All of these plasmids showed substantially reduced expression levels compared to that of pSVF8-92E. Interestingly, though, the ratio of RIA to Coatest activity for pSVF8-160 and pSVF8-170 is about 1.8, compared to 7.2 for pSVF8-92E. This result suggests that these longer heavy chain derivatives have a higher specific activity, that is, they are more efficiently assembled into active subunit complexes than the 92kD molecule itself. Also, the ratio of coagulation activity of Coatest activity is lower for the longer heavy chains at ~1.7 compared to 2.3 for 92kD and 1.35 for the complete molecule, suggesting that these longer polypeptides form complexes which are not as activated as that of the 92kD + 80kD complex.

Example 4

This example describes the preparation of stable CHO cell lines that produce the Factor VIII:C 92kD-80kD chain complex.

CHO-DUKX-B1 cells (Urlaub and Chasin, P.N.A.S. (1980) 77:4216-4220) were cotransfected with pSVF8-92C or pSVF8-92E, pSVF8-80 and pAd-DHFR using the calcium phosphate precipitation method of Graham and Van der Eb (op. cit.) and modifications described by Wigler et al. Cell (1978) 14:725-731) and Lewis et al. Somatic Cell Genetics (1980) 6:333-347. The plasmid pAd-DHFR, bearing the dhfr gene, was constructed by fusing the major late promoter from adenovirus-2 (Ad-MLP, map units 16-17.3) to the mouse dhfr cDNA at the 5′ end. DNA coding for the intron for SV40 small t antigen and the SV40 early region polyadenylation site was obtained from pSV2-neo (Southern and Berg, J. Mol. Appl. Genet. (1982) 1:327-341) and fused to the 3′ end of the dhfr cDNA. These three segments were subcloned into pBR322 to obtain plasmid pAd-DHFR. The weight ratio of plasmids were, respectively, 10:1:5 in the pSVF8-92C co-transfection and 1:1:1 or 10:10:1 in the pSVF8-92E co-transfections. In one of the pSVF8-92C transfections, the plasmids were digested with PvuI, which cuts uniquely to the beta-lactamase gene of pBR322. In that instance, linear DNA molecules were introduced instead of supercoiled molecules. Media from the resulting DHFR positive clones were screened by ELISA and Coatest, as described above. The stability of positive clones was evaluated by repeated passage in T-75 flasks and assay of the media. Table 4 below reports the expression levels for the positive, stable clones from the linear DNA.

## Table 4

| Clone | mU Coatest |
|---|---|
| **pSVF8-92C** | |
| 11-D6 | 43 |
| 11-D5 | 30 |
| **pSVF8-92E** | |
| 8-C1 | 18.2 |
| 10-C2 | 70.0 |

Primary clones from the pSVF8-92C group were carried in T-75 flasks. Clone 11-D5 proved to be the highest expressing clone, producing >90 mU/ml Coatest activity in T-75 flask cultures. After six weeks passage, this clone was put under selection in 0.025 $\mu$M, 0.05 $\mu$M, 0.1 $\mu$M, and 0.2 $\mu$M methotrexate. Clones appeared on the 0.025 uM plates, indicating that the DHFR genes have amplified at a low level of methotrexate.

Lines 8-C1 and 10-C2 of the pSVF8-92E group were selected in methotrexate (0.025 $\mu$M to 0.2 $\mu$M) and resistant clones were assayed for Factor VIII:C activity by Coatest, RIA and ELISA. Twenty-two methotrexate-resistant 8-C1 clones were examined, the data for 10 of which are reported in Table 5. The amount of Factor VIII:C amplification varies among clones, suggesting that either one of the subunit genes may have been co-amplified with the DHFR cassette, or both of them, or neither one. Note clones 8C1-A2, 8C1-A2, 8C1-C2, and 8C1-C5 as examples of these four possibilities. Similarly. 30 methotrexate-selected derivatives of 10-C2 were evaluated, the data for 20 of which are also represented in Table 5. These also contain a spectrum of activity. Note clones 10C2-A2, 10C2-D2, 10C2-B5, and 10C2-C6 as examples of the four different co-amplification possibilities.

## Table 5

| Clone | conc. MTX ($\mu$U) | Coatest ($\mu$U/ml) | ELISA ($\mu$U/ml) | RIA ($\mu$U/ml) |
|---|---|---|---|---|
| 8-C1 | 0 | 18 | 1275 | n.d. |
| 8C1-A1 | 0.1 | <50 | 1750 | 80 |
| 8C1-A2 | 0.1 | 60 | 1950 | >1000 |
| 8C1-A5 | 0.05 | 2 | 100 | 10 |
| 8C1-B3 | 0.025 | 33 | 1950 | 1000 |
| 8C1-B4 | 0.025 | 50 | 3550 | 820 |
| 8C1-B5 | 0.025 | 35 | 1950 | >1000 |
| 8C1-C2 | 0.025 | 130 | 13,100 | >>1000 |
| 8C1-C3 | 0.025 | 165 | 3900 | >>>1000 |
| 8C1-C5 | 0.025 | 30 | 1750 | 760 |
| 10-C2 | 0 | 200 | 1400 | 700 |
| 10C2-A1 | 0.05 | 61 | 1600 | 400 |
| 10C2-A2 | 0.1 | 67 | 6700 | 700 |
| 10C2-A4 | 0.05 | 63 | 2250 | 1200 |
| 10C2-A5 | 0.05 | 183 | 9450 | 2660 |
| 10C2-A6 | 0.05 | 320 | 8600 | 7400 |
| 10C2-B1 | 0.05 | 408 | 8100 | 4300 |
| 10C2-B3 | 0.05 | 134 | 800 | 9800 |
| 10C2-B4 | 0.05 | 394 | 18,000 | 7800 |
| 10C2-B5 | 0.05 | 461 | 15,000 | 8400 |
| 10C2-B6 | 0.05 | 247 | 2200 | 9800 |
| 10C2-C1 | 0.1 | 160 | 8100 | 7600 |
| 10C2-C2 | 0.05 | 228 | 6000 | 5600 |
| 10C2-C3 | 0.05 | 294 | 14,850 | 2650 |
| 10C2-C5 | 0.05 | 294 | 12,400 | 5400 |
| 10C2-C6 | 0.05 | 100 | 1350 | 520 |
| 10C2-D2 | 0.05 | 496 | 1560 | 16,400 |
| 10C2-D3 | 0.05 | 242 | 10,200 | 2260 |
| 10C2-D4 | 0.05 | 165 | 14,100 | 3500 |
| 10C2-D5 | 0.05 | 316 | 7800 | 5200 |
| 10C2-D6 | 0.05 | 141 | 1600 | 6400 |

n.d. = not determined

Plasmids pSVF8-92 and pSVF8-80 were deposited at the American Type Culture Collection (ATCC) on 24 January 1986 and given ATCC Accession Nos. 40222 and 40223, respectively. Plasmid pSVF8-200 was deposited at the ATCC on 17 July 1985 and was given ATCC Accession No. 40190.

Although the foregoing invention has been described in some detail by way of illustration and example for purposes of clarity of understanding, it will be obvious that certain changes and modifications may be practiced within the scope of the appended claims.

## Claims

**Claims for the following Contracting States : AT, BE, CH, DE, FR, GB, GR, IT, LI, LU, NL, SE**

1. A method of producing a recombinant protein complex having human Factor VIII:C activity but lacking all or part of the B domain of human Factor VIII:C comprising
   - co-expressing in trans in a eukaryotic transformant host cell
     (a) a first gene encoding a signal sequence and a polypeptide having the amino acid sequence of the A domain of human Factor VIII:C or a sequence which differs therefrom by not more than 10 % amino acid substitutions, and optionally including an N-terminal portion of the B domain of human Factor VIII:C, and

EP 0 232 112 B1

(b) a second gene encoding a signal sequence and a polypeptide having the amino acid sequence of the C domain of human Factor VIII:C or a sequence which differs therefrom by not more than 10 % amino acid substitutions, and

- secreting the recombinant protein complex.

2. The method according to claim 1, wherein not more than about 5 number-% of the amino acids of said amino acid sequences differ from the naturally occuring amino acid sequences of the Factor VIII:C A and C domains.

3. The method according to any of claims 1 and 2, wherein the amino acid sequence of the polypeptide encoded by the second gene is the same as the amino acid sequence of amino acids 1649-2322 of human Factor VIII:C.

4. The method according to any of claims 1 to 3, wherein the amino acid sequence of the polypeptide encoded by the first gene is the same as the amino acid sequence of amino acids 1-740 of human Factor VIII:C.

5. The method according to any of claims 1 to 3, wherein the amino acid sequence of the polypeptide encoded by the first gene is the same as the amino acid sequence of amino acids 1-1102 of human Factor VIII:C.

6. The method according to any of claims 1 to 3, wherein the amino acid sequence of the polypeptide encoded by the first gene is the same as the amino acid sequence of amino acids 1-1315 of human Factor VIII:C.

7. The method according to any of claims 1 to 3, wherein the amino acid sequence of the polypeptide encoded by the first gene is the same as the amino acid sequence of amino acids 1-1405 of human Factor VIII:C.

8. The method according to any of claims 1 to 7, wherein the eukaryotic transformant host cell is a mammalian cell.

9. The method according to any of claims 1 to 8, wherein the first gene includes a 5'-untranslated DNA sequence of human Factor VIII:C that enhances the expression of the polypeptide encoded by the first gene, or the first gene includes a 3'-untranslated DNA sequence of human Factor VIII:C that enhances the expression of the polypeptide encoded by the first gene.

10. The method according to any of claims 1 to 9, wherein the first gene and the second gene are in separate expression plasmids.

11. The use of a first gene encoding a polypeptide having the amino acid sequence of the A domain of human Factor VIII:C, and of a second gene encoding a polypeptide having the amino sequence of the C domain of human Factor VIII:C, or of first and second genes encoding amino acid sequences differing from the above indicated sequences by not more than 10 % amino acid substitutions, each of said genes having an independent signal sequence, in an expression system for producing a protein complex having Factor VIII:C activity.

12. A DNA composition comprising first and second expression cassettes, said first expression cassette comprising a first gene encoding a signal sequence and a polypeptide comprising the amino acid sequence of the A domain of human Factor VIII:C or a sequence which differs therefrom by not more than 10 % amino acid substitutions, and optionally an N-terminal portion of the B domain of human Factor VIII:C under transcriptional and translational regulatory signals functional in a host cell, and said second expression cassette comprising a second gene encoding a signal sequence and a polypeptide comprising the amino acid sequence of the C-domain of human Factor VIII:C or a sequence which differs therefrom by not more than 10 % amino acid substitutions, and not including the B domain under transcriptional and translational regulatory signals functional in said host cell.

16

**13.** The DNA composition according to claim 12, wherein said first cassette gene encodes a polypeptide comprising at least about 90 % of the amino acid sequence of human Factor VIII:C amino acids 1-740, and said second cassette gene encodes a polypeptide comprising at least about 90 % of the amino acid sequence of human Factor VIII:C amino acids 1649-2332.

**14.** A host mammalian cell containing a DNA composition according to claim 12 or 13.

**Claims for the following Contracting State : ES**

**1.** A method of producing a recombinant protein complex having human Factor VIII:C activity but lacking all or part of the B domain of human Factor VIII:C comprising
- co-expressing in trans in a eukaryotic transformant host cell
(a) a first gene encoding a signal sequence and a polypeptide having the amino acid sequence of the A domain of human Factor VIII:C or a sequence which differs therefrom by not more than 10 % amino acid substitutions, and optionally including an N-terminal portion of the B domain of human Factor VIII:C, and
(b) a second gene encoding a signal sequence and a polypeptide having the amino acid sequence of the C domain of human Factor VIII:C or a sequence which differs therefrom by not more than 10 % amino acid substitutions,
and
- secreting the recombinant protein complex.

**2.** The method according to claim 1, wherein not more than about 5 number-% of the amino acids of said amino acid sequences differ from the naturally occuring amino acid sequences of the Factor VIII:C A and C domains.

**3.** The method according to any of claims 1 and 2, wherein the amino acid sequence of the polypeptide encoded by the second gene is the same as the amino acid sequence of amino acids 1649-2322 of human Factor VIII:C.

**4.** The method according to any of claims 1 to 3, wherein the amino acid sequence of the polypeptide encoded by the first gene is the same as the amino acid sequence of amino acids 1-740 of human Factor VIII:C.

**5.** The method according to any of claims 1 to 3, wherein the amino acid sequence of the polypeptide encoded by the first gene is the same as the amino acid sequence of amino acids 1-1102 of human Factor VIII:C.

**6.** The method according to any of claims 1 to 3, wherein the amino acid sequence of the polypeptide encoded by the first gene is the same as the amino acid sequence of amino acids 1-1315 of human Factor VIII:C.

**7.** The method according to any of claims 1 to 3, wherein the amino acid sequence of the polypeptide encoded by the first gene is the same as the amino acid sequence of amino acids 1-1405 of human Factor VIII:C.

**8.** The method according to any of claims 1 to 7, wherein the eukaryotic transformant host cell is a mammalian cell.

**9.** The method according to any of claims 1 to 8, wherein the first gene includes a 5'-untranslated DNA sequence of human Factor VIII:C that enhances the expression of the polypeptide encoded by the first gene, or the first gene includes a 3'-untranslated DNA sequence of human Factor VIII:C that enhances the expression of the polypeptide encoded by the first gene.

**10.** The method according to any of claims 1 to 9, wherein the first gene and the second gene are in separate expression plasmids.

**11.** The method according to any of claims 1 to 10, wherein a host cell is used for the co-expression of the first gene and the second gene, which has been transformed with use of a first and a second expression cassette, said first expression cassette comprising a first gene encoding a signal sequence and a polypeptide comprising the amino acid sequence of the A domain of human Factor VIII:C or a sequence which differs therefrom by not more than 10 % amino acid substitutions, and optionally an N-terminal portion of the B domain of human Factor VIII:C under transcriptional and translational regulatory signals functional in a host cell, and said second expression cassette comprising a second gene encoding a signal sequence and a polypeptide comprising the amino acid sequence of the C-domain of human Factor VIII:C or a sequence which differs therefrom by not more than 10 % amino acid substitutions, and not including the B domain under transcriptional and translational regulatory signals functional in said host cell.

**12.** The method according to claim 11, wherein said first cassette gene encodes a polypeptide comprising at least about 90 % of the amino acid sequence of human Factor VIII:C amino acids 1-740, and said second cassette gene encodes a polypeptide comprising at least about 90 % of the amino acid sequence of human Factor VIII:C amino acids 1649-2332.

**13.** The use of a first gene encoding a polypeptide having the amino acid sequence of the A domain of human Factor VIII:C, and of a second gene encoding a polypeptide having the amino sequence of the C domain of human Factor VIII:C, or of first and second genes encoding amino acid sequences differing from the above indicated sequences by not mare than 10 % amino acid substitutions, each of said genes having an independent signal sequence, in an expression system for producing a protein complex having Factor VIII:C activity.

**Patentansprüche**

**Patentansprüche für folgende Vertragsstaaten : AT, BE, CH, DE, FR, GB, GR, IT, LI, LU, NL, SE**

**1.** Verfahren zur Herstellung eines rekombinanten Proteinkomplexes mit Humanfaktor-VIII:C-Aktivität, der die gesamte B-Domäne von Humanfaktor VIII:C oder einen Teil davon nicht aufweist,
mit folgenden Schritten:
- Trans-Coexpression
(a) eines ersten Gens, das eine Signalsequenz und ein Polypeptid codiert, das die Aminosäuresequenz der A-Domäne von Humanfaktor VIII:C oder eine Sequenz, die sich davon um nicht mehr als 10 % Aminosäuresubstitutionen unterscheidet, und wahlweise einen N-terminalen Teil der B-Domäne von Humanfaktor VIII:C umfaßt, und
(b) eines zweiten Gens, das eine Signalsequenz und ein Polypeptid codiert, das die Aminosäuresequenz der C-Domäne von Humanfaktor VIII:C oder eine Sequenz umfaßt, die sich davon um nicht mehr als 10 % Aminosäuresubstitutionen unterscheidet,
in einer transformierten eukaryontischen Wirtzelle und
- Sekretion des rekombinanten Proteinkomplexes.

**2.** Verfahren nach Anspruch 1, wobei nicht mehr als etwa 5 % der Gesamtzahl der Aminosäuren der Aminosäuresequenzen von den natürlicherweise vorliegenden Aminosäuresequenzen der A- und C-Domäne von Faktor VIII:C verschieden sind.

**3.** Verfahren nach Anspruch 1 oder 2, wobei die Aminosäuresequenz des vom zweiten Gen codierten Polypeptids die gleiche ist wie die Aminosäuresequenz der Aminosäuren 1649-2322 von Humanfaktor VIII:C.

**4.** Verfahren nach einem der Ansprüche 1 bis 3, wobei die Aminosäuresequenz des vom ersten Gen codierten Polypeptids die gleiche ist wie die Aminosäuresequenz der Aminosäuren 1-740 von Humanfaktor VIII:C.

**5.** Verfahren nach einem der Ansprüche 1 bis 3, wobei die Aminosäuresequenz des vom ersten Gen codierten Polypeptids die gleiche ist wie die Aminosäuresequenz der Aminosäuren 1-1102 von Humanfaktor VIII:C.

6. Verfahren nach einem der Ansprüche 1 bis 3, wobei die Aminosäuresequenz des vom ersten Gen codierten Polypeptids die gleiche ist wie die Aminosäuresequenz der Aminosäuren 1-1315 von Humanfaktor VIII:C.

7. Verfahren nach einem der Ansprüche 1 bis 3, wobei die Aminosäuresequenz des vom ersten Gen codierten Polypeptids die gleiche ist wie die Aminosäuresequenz der Aminosäuren 1-1405 von Humanfaktor VIII:C.

8. Verfahren nach einem der Ansprüche 1 bis 7, wobei die transformierte eukaryontische Wirtzelle eine Säugerzelle ist.

9. Verfahren nach einem der Ansprüche 1 bis 8, wobei das erste Gen eine 5'-nichttranslatierte DNA-Sequenz von Humanfaktor VIII:C, welche für die Expression des vom ersten Gen codierten Polypeptids als Enhancer wirkt, oder eine 3'-nichttranslatierte DNA-Sequenz von Humanfaktor VIII:C aufweist, welche für die Expression des vom ersten Gen codierten Polypeptids als Enhancer wirkt.

10. Verfahren nach einem der Ansprüche 1 bis 9, wobei das erste Gen und das zweite Gen in separaten Expressionsplasmiden vorliegen.

11. Verwendung eines ersten Gens, das ein Polypeptid mit der Aminosäuresequenz der A-Domäne von Humanfaktor VIII:C codiert, und eines zweiten Gens, das ein Polypeptid mit der Aminosäuresequenz der C-Domäne von Humanfaktor VIII:C codiert, oder eines ersten und eines zweiten Gens, die Aminosäuresequenzen codieren, die sich von den vorstehend angegebenen Sequenzen um nicht mehr als 10 % Aminosäuresubstitutionen unterscheiden, wobei die Gene eine unabhängige Signalsequenz aufweisen, in einem Expressionssystem zur Herstellung eines Proteinkomplexes mit Faktor-VIII:C-Aktivität.

12. DNA-Zusammensetzung, die eine erste und eine zweite Expressionskassette aufweist, wobei
die erste Expressionskassette ein erstes Gen aufweist, das eine Signalsequenz und ein Polypeptid unter der Wirkung von Regulationssignalen, die in einer Wirtzelle wirksam sind, codiert, das die Aminosäuresequenz der A-Domäne von Humanfaktor VIII:C oder eine Sequenz, die sich davon um nicht mehr als 10 % Aminosäuresubstitutionen unterscheidet, und wahlweise einen N-terminalen Teil der B-Domäne von Humanfaktor VIII:C umfaßt,
und
die zweite Expressionskassette ein zweites Gen umfaßt, das eine Signalsequenz und ein Polypeptid unter der Wirkung von Regulationssignalen, die in der Wirtzelle wirksam sind, codiert, das die Aminosäuresequenz der C-Domäne von Humanfaktor VIII:C oder eine Sequenz umfaßt, die sich davon um nicht mehr als 10 % Aminosäuresubstitutionen unterscheidet.

13. DNA-Zusammensetzung nach Anspruch 12, wobei das Gen der ersten Kassette ein Polypeptid codiert, das mindestens etwa 90 % der Aminosäuren 1-740 der Aminosäuresequenz von Humanfaktor VIII:C codiert, und das Gen der zweiten Kassette ein Polypeptid codiert, das mindestens etwa 90 % der Aminosäuren 1649-2332 der Aminosäuresequenz von Humanfaktor VIII:C codiert.

14. Säuger-Wirtzelle, die eine DNA-Zusammensetzung nach Anspruch 12 oder 13 enthält.

**Patentansprüche für folgenden Vertragsstaat : ES**

1. Verfahren zur Herstellung eines rekombinanten Proteinkomplexes mit Humanfaktor-VIII:C-Aktivität, der die gesamte B-Domäne von Humanfaktor VIII:C oder einen Teil davon nicht aufweist,
mit folgenden Schritten:
- Trans-Coexpression
(a) eines ersten Gens, das eine Signalsequenz und ein Polypeptid codiert, das die Aminosäuresequenz der A-Domäne von Humanfaktor VIII:C oder eine Sequenz, die sich davon um nicht mehr als 10 % Aminosäuresubstitutionen unterscheidet, und wahlweise einen N-terminalen Teil der B-Domäne von Humanfaktor VIII:C umfaßt, und
(b) eines zweiten Gens, das eine Signalsequenz und ein Polypeptid codiert, das die Aminosäuresequenz der C-Domäne von Humanfaktor VIII:C oder eine Sequenz umfaßt, die sich davon um nicht mehr als 10 % Aminosäuresubstitutionen unterscheidet,
in einer transformierten eukaryontischen Wirtzelle und
- Sekretion des rekombinanten Proteinkomplexes.

2. Verfahren nach Anspruch 1, wobei nicht mehr als etwa 5 % der Gesamtzahl der Aminosäuren der Aminosäuresequenzen von den natürlicherweise vorliegenden Aminosäuresequenzen der A- und C-Domäne von Faktor VIII:C verschieden sind.

3. Verfahren nach Anspruch 1 oder 2, wobei die Aminosäuresequenz des vom zweiten Gen codierten Polypeptids die gleiche ist wie die Aminosäuresequenz der Aminosäuren 1649-2322 von Humanfaktor VIII:C.

4. Verfahren nach einem der Ansprüche 1 bis 3, wobei die Aminosäuresequenz des vom ersten Gen codierten Polypeptids die gleiche ist wie die Aminosäuresequenz der Aminosäuren 1-740 von Humanfaktor VIII:C.

5. Verfahren nach einem der Ansprüche 1 bis 3, wobei die Aminosäuresequenz des vom ersten Gen codierten Polypeptids die gleiche ist wie die Aminosäuresequenz der Aminosäuren 1-1102 von Humanfaktor VIII:C.

6. Verfahren nach einem der Ansprüche 1 bis 3, wobei die Aminosäuresequenz des vom ersten Gen codierten Polypeptids die gleiche ist wie die Aminosäuresequenz der Aminosäuren 1-1315 von Humanfaktor VIII:C.

7. Verfahren nach einem der Ansprüche 1 bis 3, wobei die Aminosäuresequenz des vom ersten Gen codierten Polypeptids die gleiche ist wie die Aminosäuresequenz der Aminosäuren 1-1405 von Humanfaktor VIII:C.

8. Verfahren nach einem der Ansprüche 1 bis 7, wobei die transformierte eukaryontische Wirtzelle eine Säugerzelle ist.

9. Verfahren nach einem der Ansprüche 1 bis 8, wobei das erste Gen eine 5'-nichttranslatierte DNA-Sequenz von Humanfaktor VIII:C, welche für die Expression des vom ersten Gen codierten Polypeptids als Enhancer wirkt, oder eine 3'-nichttranslatierte DNA-Sequenz von Humanfaktor VIII:C aufweist, welche für die Expression des vom ersten Gen codierten Polypeptids als Enhancer wirkt.

10. Verfahren nach einem der Ansprüche 1 bis 9, wobei das erste Gen und das zweite Gen in separaten Expressionsplasmiden vorliegen.

11. Verfahren nach einem der Ansprüche 1 bis 10, wobei eine Wirtzelle für die Coexpression des ersten und des zweiten Gens verwendet wird, die unter Verwendung einer ersten und einer zweiten Expressionskassette transformiert wurde, wobei
die erste Expressionskassette ein erstes Gen aufweist, das eine Signalsequenz und ein Polypeptid unter der Wirkung von Regulationssignalen, die in einer Wirtzelle wirksam sind, codiert, das die Aminosäuresequenz der A-Domäne von Humanfaktor VIII:C oder eine Sequenz, die sich davon um nicht mehr als 10 % Aminosäuresubstitutionen unterscheidet, und wahlweise einen N-terminalen Teil der B-Domäne von Humanfaktor VIII:C umfaßt,
und
die zweite Expressionskassette ein zweites Gen umfaßt, das eine Signalsequenz und ein Polypeptid unter der Wirkung von Regulationssignalen, die in der Wirtzelle wirksam sind, codiert, das die Aminosäuresequenz der C-Domäne von Humanfaktor VIII:C oder eine Sequenz umfaßt, die sich davon um nicht mehr als 10 % Aminosäuresubstitutionen unterscheidet.

12. Verfahren nach Anspruch 11, wobei das Gen der ersten Kassette ein Polypeptid codiert, das mindestens etwa 90 % der Aminosäuren 1-740 der Aminosäuresequenz von Humanfaktor VIII:C codiert, und das Gen der zweiten Kassette ein Polypeptid codiert, das mindestens etwa 90 % der Aminosäuren 1649-2332 der Aminosäuresequenz von Humanfaktor VIII:C codiert.

**13.** Verwendung eines ersten Gens, das ein Polypeptid mit der Aminosäuresequenz der A-Domäne von Humanfaktor VIII:C codiert, und eines zweiten Gens, das ein Polypeptid mit der Aminosäuresequenz der C-Domäne von Humanfaktor VIII:C codiert, oder eines ersten und eines zweiten Gens, die Aminosäuresequenzen codieren, die sich von den vorstehend angegebenen Sequenzen um nicht mehr als 10 % Aminosäuresubstitutionen unterscheiden, wobei die Gene eine unabhängige Signalsequenz aufweisen, in einem Expressionssystem zur Herstellung eines Proteinkomplexes mit Faktor-VIII:C-Aktivität.

**Revendications**

**Revendications pour les Etats contractants suivants : AT, BE, CH, DE, FR, GB, GR, IT, LI, LU, NL, SE**

**1.** Procédé de production d'un complexe protéique recombiné ayant l'activité du facteur VIII:C humain mais dépourvu de tout ou partie du domaine B du facteur VIII:C humain, comprenant
   - la co-expression en trans dans une cellule hôte eucaryote transformée
   (a) d'un premier gène codant une séquence signal et un polypeptide ayant la séquence d'acides aminés du domaine A du facteur VIII:C humain ou une séquence qui n'en diffère pas par plus de 10% de substitutions d'acides aminés, et comprenant éventuellement une partie N-terminale du domaine B du facteur VIII:C humain, et
   (b) un second gène codant une séquence signal et un polypeptide ayant la séquence d'acides aminés du domaine C du facteur VIII:C humain ou une séquence qui n'en diffère pas par plus de 10% de substitutions d'acides aminés, et
   - la sécrétion du complexe protéique recombiné.

**2.** Procédé selon la revendication 1, dans lequel pas plus d'environ 5% en noire des acides aminés desdites séquences d'acides aminés diffèrent des séquences d'acides aminés naturelles des domaines A et C du facteur VIII:C.

**3.** Procédé selon l'une quelconque des revendications 1 et 2, dans lequel la séquence d'acides aminés du polypeptide codé par le second gène est la même que la séquence d'acides aminés des acides aminés 1649 à 2322 du facteur VIII:C humain.

**4.** Procédé selon l'une quelconque des revendications 1 à 3, dans lequel la séquence d'acides aminés du polypeptide codé par le premier gène est la même que la séquence d'acides aminés des acides aminés 1 à 740 du facteur VIII:C humain.

**5.** Procédé selon l'une quelconque des revendications 1 à 3, dans lequel la séquence d'acides aminés du polypeptide codé par le premier gène est la même que la séquence d'acides aminés des acides aminés 1 à 1102 du facteur VIII:C humain.

**6.** Procédé selon l'une quelconque des revendications 1 à 3, dans lequel la séquence d'acides aminés du polypeptide codé par le premier gène est la même que la séquence d'acides aminés des acides aminés 1 à 1315 du facteur VIII:C humain.

**7.** Procédé selon l'une quelconque des revendications 1 à 3, dans lequel la séquence d'acides aminés du polypeptide codé par le premier gène est la même que la séquence d'acides aminés des acides aminés 1 à 1405 du facteur VIII:C humain.

**8.** Procédé selon l'une quelconque des revendications 1 à 7, dans lequel la cellule hôte eucaryote transformée est une cellule de mammifère.

**9.** Procédé selon l'une quelconque des revendications 1 à 8, dans lequel le premier gène comprend une séquence d'ADN non traduite en 5' du facteur VIII:C humain qui amplifie l'expression du polypeptide codé par le premier gène, ou bien le premier gène comprend une séquence d'ADN non traduite en 5' du facteur VIII:C humain qui amplifie l'expression du polypeptide codé par le premier gène.

**10.** Procédé selon l'une quelconque des revendications 1 à 9, dans lequel le premier gène et le second gène sont dans des plasmides d'expression séparés.

**11.** Utilisation d'un premier gène codant un polypeptide ayant la séquence d'acides aminés du domaine A du facteur VIII:C humain et d'un second gène codant un polypeptide ayant la séquence d'acides aminés du domaine C du facteur VIII:C humain, ou d'un premier et d'un second gène codant des séquences d'acides aminés qui ne diffèrent pas des séquences indiquées ci-dessus par plus de 10% de substitutions d'acides aminés, chacun desdits gènes ayant une séquence signal indépendante, dans un système d'expression pour produire un complexe protéique ayant l'activité du facteur VIII:C.

**12.** Composition d'ADN comprenant des première et seconde cassettes d'expression, ladite première cassette d' expression comprenant un premier gène codant une séquence signal et un polypeptide comprenant la séquence d'acides aminés du domaine A du facteur VIII:C humain ou une séquence qui n'en diffère pas par plus de 10% de substitutions d'acides aminés, et éventuellement une partie N-terminale du domaine B du facteur VIII:C humain sous des signaux de régulation de la transcription et de la traduction fonctionnels dans une cellule hôte, et ladite seconde cassette d'expression comprenant un second gène codant une séquence signal et un polypeptide comprenant la séquence d'acides aminés du domaine C du facteur VIII:C humain ou une séquence qui n'en diffère pas par plus de 10% de substitutions d'acides aminés, et ne comprenant pas le domaine B sous des signaux de régulation de la transcription et de la traduction fonctionnels dans ladite cellule hôte.

**13.** Composition d'ADN selon la revendication 12, dans laquelle ledit gène de la première cassette code un polypeptide comprenant au moins environ 90% de la séquence d'acides aminés des acides aminés 1 à 740 du facteur VIII:C humain, et ledit gène de la seconde cassette code un polypeptide comprenant au moins environ 90% de la séquence d'acides aminés des acides aminés 1649 à 2332 du facteur VIII:C humain.

**14.** Cellule hôte de mammifère contenant une composition d'ADN selon la revendication 12 ou 13.

**Revendications pour l'Etat contractant suivant : ES**

**1.** Procédé de production d'un complexe protéique recombiné ayant l'activité du facteur VIII:C humain mais dépourvu de tout ou partie du domaine B du facteur VIII:C humain, comprenant
- la co-expression en trans dans une cellule hôte eucaryote transformée
(a) d'un premier gène codant une séquence signal et un polypeptide ayant la séquence d'acides aminés du domaine A du facteur VIII:C humain ou une séquence qui n'en diffère pas par plus de 10% de substitutions d'acides aminés, et comprenant éventuellement une partie N-terminale du domaine B du facteur VIII:C humain, et
(b) un second gène codant une séquence signal et un polypeptide ayant la séquence d'acides aminés du domaine C du facteur VIII:C humain ou une séquence qui n'en diffère pas par plus de 10% de substitutions d'acides aminés, et
- la sécrétion du complexe protéique recombiné.

**2.** Procédé selon la revendication 1, dans lequel pas plus d'environ 5% en nombre des acides aminés desdites séquences d'acides aminés diffèrent des séquences d'acides aminés naturelles des domaines A et C du facteur VIII:C.

**3.** Procédé selon l'une quelconque des revendications 1 et 2, dans lequel la séquence d'acides aminés du polypeptide codé par le second gène est la même que la séquence d'acides aminés des acides aminés 1649 à 2322 du facteur VIII:C humain.

**4.** Procédé selon l'une quelconque des revendications 1 à 3, dans lequel la séquence d'acides aminés du polypeptide codé par le premier gène est la même que la séquence d'acides aminés des acides aminés 1 à 740 du facteur VIII:C humain.

**5.** Procédé selon l'une quelconque des revendications 1 à 3, dans lequel la séquence d'acides aminés du polypeptide codé par le premier gène est la même que la séquence d'acides aminés des acides aminés 1 à 1102 du facteur VIII:C humain.

**6.** Procédé selon l'une quelconque des revendications 1 à 3, dans lequel la séquence d'acides aminés du polypeptide codé par le premier gène est la même que la séquence d'acides aminés des acides aminés 1 à 1315 du facteur VIII:C humain.

7. Procédé selon l'une quelconque des revendications 1 à 3, dans lequel la séquence d'acides aminés du polypeptide codé par le premier gène est la même que la séquence d'acides aminés des acides aminés 1 à 1405 du facteur VIII:C humain.

8. Procédé selon l'une quelconque des revendications 1 à 7, dans lequel la cellule hôte eucaryote transformée est une cellule de mammifère.

9. Procédé selon l'une quelconque des revendications 1 à 8, dans lequel le premier gène comprend une séquence d'ADN non traduite en 5' du facteur VIII:C humain qui amplifie l'expression du polypeptide codé par le premier gène, ou bien le premier gène comprend une séquence d'ADN non traduite en 5' du facteur VIII:C humain qui amplifie l'expression du polypeptide codé par le premier gène.

10. Procédé selon l'une quelconque des revendications 1 à 9, dans lequel le premier gène et le second gène sont dans des plasmides d'expression séparés.

11. Procédé selon l'une quelconque des revendications 1 à 10, dans lequel une cellule hôte est utilisée pour la co-expression du premier gène et du second gène, qui a été transformée au moyen d'une première et d'une seconde cassette d'expression, ladite première cassette d'expression comprenant un premier gène codant une séquence signal et un polypeptide comprenant la séquence d'acides aminés du domaine A du facteur VIII:C humain ou une séquence qui n'en diffère pas par plus de 10% de substitutions d'acides aminés, et éventuellement une partie N-terminale du domaine B du facteur VIII:C humain sous des signaux de régulation de la transcription et de la traduction fonctionnels dans une cellule hôte, et ladite seconde cassette d'expression comprenant un second gène codant une séquence signal et un polypeptide comprenant la séquence d'acides aminés du domaine C du facteur VIII:C humain ou une séquence qui n'en diffère pas par plus de 10% de substitutions d'acides aminés, et ne comprenant pas le domaine B sous des signaux de régulation de la transcription et de la traduction fonctionnels dans ladite cellule hôte.

12. Procédé selon la revendication 11, dans lequel ledit gène de la première cassette code un polypeptide comprenant au moins environ 90% de la séquence d'acides aminés des acides aminés 1 à 740 du facteur VIII:C humain, et ledit gène de la seconde cassette code un polypeptide comprenant au moins environ 90% de la séquence d'acides aminés des acides aminés 1649 à 2332 du facteur VIII:C humain.

13. Utilisation d'un premier gène codant un polypeptide ayant la séquence d'acides aminés du domaine A du facteur VIII:C humain et d'un second gène codant un polypeptide ayant la séquence d'acides aminés du domaine C du facteur VIII:C humain, ou d'un premier et d'un second gène codant des séquences d'acides aminés qui ne diffèrent pas des séquences indiquées ci-dessus par plus de 10% de substitutions d'acides aminés, chacun desdits gènes ayant une séquence signal indépendante, dans un système d'expression pour produire un complexe protéique ayant l'activité du facteur VIII:C.

FIG. IA

FIG. IB

FIG. IC

FIG. ID

FIG. IE